# EUROPEAN PATENT APPLICATION

(11) **EP 4 776 223 A1**
(43) Date of publication of application: **15.07.2026**
(21) Application number: 25224091.6
(22) Date of filing: 16.12.2025
(51) Int. Cl.: G06T 7/33, A61B 8/00

(54) **IMAGE PROCESSING APPARATUS, OPERATION METHOD OF IMAGE PROCESSING APPARATUS, AND OPERATION PROGRAM OF IMAGE PROCESSING APPARATUS**

(30) Priority: 08.01.2025 JP 2025003056
(71) Applicant: FUJIFILM Corporation, Tokyo 106-8620 (JP)
(72) Inventor: GOTO, Tsubasa, Tokyo, 106-8620 (JP)
(74) Representative: Dehns Germany Partnerschaft mbB

(57) **Abstract**

Provided are an image processing apparatus, an operation method of the image processing apparatus, and an operation program of the image processing apparatus that can perform registration of an ultrasound image (13) and a three-dimensional image such that a registration result does not fall into a local solution and a clearly incorrect registration result is not output.

An acquisition unit acquires a CT image of a patient. A generation unit acquires a US image of the patient by generating the US image from an echo signal. A selection unit repeatedly selects a plurality of reference points from a first point group of the US image and a plurality of target points from a second point group of the CT image a plurality of times. A derivation unit derives a candidate parameter each time the reference point and the target point are selected. A determination unit determines whether the candidate parameter satisfies a constraint condition related to a position and a posture of an ultrasound probe (10) at the time of capturing the US image.

## Description

### BACKGROUND OF THE INVENTION

### 1. Field of the Invention

The technology of the present disclosure relates to an image processing apparatus, an operation method of an image processing apparatus, and an operation program of an image processing apparatus.

### 2. Description of the Related Art

For example, a technique of performing display for supporting an operator by performing registration of an ultrasound image captured by bringing an ultrasound probe into contact with a body surface of a subject with a three-dimensional image such as a computed tomography (CT) image or a magnetic resonance imaging (MRI) image captured by imaging the subject in a CT apparatus or an MRI apparatus before surgery, and displaying a target site of surgery such as a tumor as a marker in intraoperative radiofrequency ablation (RFA) is known.

Depending on an accuracy of the registration of the ultrasound image and the three-dimensional image, a clearly incorrect registration result may be output. Therefore, in US2014/0193053A, in a case in which a rigid transformation parameter that is clearly incorrect, such as a parameter that sets a position of the ultrasound probe inside an organ such as a liver, is derived in deriving the rigid transformation parameter for registration, a high penalty is imposed. In the technique described in US2014/0193053A, an iterative closest point (ICP) algorithm is used as a registration method.

### SUMMARY OF THE INVENTION

In the ICP algorithm used in the technique described in US2014/0193053A, in a case in which there is a large deviation in an initial setting of a reference point of a source image and a target point of a destination image, the ICP algorithm cannot perform accurate registration, and the rigid transformation parameter falls into a local solution.

One embodiment according to the technology of the present disclosure provides an image processing apparatus, an operation method of an image processing apparatus, and an operation program of an image processing apparatus that in a case of registering an ultrasound image with a three-dimensional image, prevents the registration result from falling into a local solution and can prevent the output of a clearly incorrect registration result.

An image processing apparatus according to the present disclosure includes a processor, in which the processor is configured to: acquire an ultrasound image of a subject and a three-dimensional image of the subject that is a target of registration of the ultrasound image; repeatedly select a plurality of reference points from a first point group of the ultrasound image and a plurality of target points from a second point group of the three-dimensional image a plurality of times; derive a candidate of a transformation parameter between the first point group and the second point group each time the reference points and the target points are selected; and determine whether the candidate satisfies a constraint condition related to a position and/or posture of an ultrasound probe at the time of capturing the ultrasound image.

It is preferable that the processor is configured to: transform the three-dimensional image using the candidate determined to satisfy the constraint condition; calculate a similarity between the ultrasound image and the transformed three-dimensional image; and not perform the transformation and the calculation with respect to the candidate determined not to satisfy the constraint condition.

It is preferable that the processor is configured to: transform the second point group using the candidate determined to satisfy the constraint condition; calculate a similarity between the first point group and the transformed second point group; and not perform the transformation and the calculation with respect to the candidate determined not to satisfy the constraint condition.

It is preferable that the processor is configured to: transform the second point group using the candidate determined to satisfy the constraint condition; extract a point that is not an outlier in the first point group based on the first point group and the transformed second point group; and not perform the transformation and the extraction on the candidate determined not to satisfy the constraint condition.

It is preferable that the processor is configured to adopt, among the plurality of candidates determined to satisfy the constraint condition, a candidate having the highest similarity as the transformation parameter.

It is preferable that the first point group and the second point group are point groups related to an organ and/or a blood vessel shown in the ultrasound image and the three-dimensional image.

It is preferable that the processor is configured to: determine whether an accuracy of the provisional transformation parameter satisfies an accuracy condition; adopt the provisional transformation parameter as the transformation parameter in a case where the accuracy of the provisional transformation parameter is determined to satisfy the accuracy condition; and determine the transformation parameters using the first point group and the second point group related to the organ in a case where the accuracy of the provisional transformation parameter is determined not to satisfy the accuracy condition.

It is preferable that the processor is configured to: determine whether the candidate satisfies a first constraint condition that is the constraint condition related to the position; and determine whether the candidate satisfies a second constraint condition that is the constraint condition related to the posture.

It is preferable that the first constraint condition is a condition related to the position and a distance to a body surface of the subject in the three-dimensional image.

It is preferable that the second constraint condition is a condition related to an orientation of the ultrasound probe with respect to the body surface of the subject in the three-dimensional image.

It is preferable that the processor is configured to: generate a distance image representing a distance from a body surface of the subject from the three-dimensional image; and determine whether the candidate satisfies the constraint condition using the distance image.

It is preferable that the constraint condition is modifiable.

An operation method of an image processing apparatus according to the present disclosure includes: acquiring an ultrasound image of a subject and a three-dimensional image of the subject that is a target of registration of the ultrasound image; repeatedly selecting a plurality of reference points from a first point group of the ultrasound image and a plurality of target points from a second point group of the three-dimensional image a plurality of times; deriving a candidate of a transformation parameter between the first point group and the second point group each time the reference points and the target points are selected; and determining whether the candidate satisfies a constraint condition related to a position and/or posture of an ultrasound probe at the time of capturing the ultrasound image.

An operation program of an image processing apparatus, the operation program causing a computer to execute a process including: acquiring an ultrasound image of a subject and a three-dimensional image of the subject that is a target of registration of the ultrasound image; repeatedly selecting a plurality of reference points from a first point group of the ultrasound image and a plurality of target points from a second point group of the three-dimensional image a plurality of times; deriving a candidate of a transformation parameter between the first point group and the second point group each time the reference points and the target points are selected; and determining whether the candidate satisfies a constraint condition related to a position and/or posture of an ultrasound probe at the time of capturing the ultrasound image.

According to the technology of the present disclosure, it is possible to provide an image processing apparatus, an operation method of an image processing apparatus, and an operation program of an image processing apparatus that in a case of registering an ultrasound image with a three-dimensional image, prevents the registration result from falling into a local solution and can prevent the output of a clearly incorrect registration result.

### BRIEF DESCRIPTION OF THE DRAWINGS

FIG. 1 is a diagram showing an intraoperative state of RFA.
FIG. 2 is an explanatory diagram of functions of an apparatus main body of an ultrasound diagnostic apparatus.
FIG. 3 is a block diagram showing a computer constituting the apparatus main body.
FIG. 4 is a block diagram showing a processing unit of a CPU of the apparatus main body.
FIG. 5 is a diagram showing processing of an extraction unit.
FIG. 6 is a diagram showing processing of the extraction unit.
FIG. 7 is a diagram showing an extraction result.
FIG. 8 is a block diagram showing a detailed configuration of an initial registration unit.
FIG. 9 is a diagram showing a distance image.
FIG. 10 is a diagram showing a point indicating a position of an ultrasound probe and a point indicating an orientation of the ultrasound probe.
FIG. 11 is a diagram showing a selection result.
FIG. 12 is a diagram showing a state in which the point indicating the position of the ultrasound probe and the point indicating the orientation of the ultrasound probe are transformed using a candidate parameter.
FIG. 13 is a diagram showing a first constraint condition and a second constraint condition.
FIG. 14 is a diagram showing a state in which it is determined whether or not the candidate parameter satisfies the first constraint condition.
FIG. 15 is a diagram showing a state in which it is determined whether or not the candidate parameter satisfies the first constraint condition.
FIG. 16 is a diagram showing a state in which it is determined whether or not the candidate parameter satisfies the second constraint condition.
FIG. 17 is a diagram showing a state in which it is determined whether or not the candidate parameter satisfies the second constraint condition.
FIGS. 18A and 18B are diagrams showing a determination result and subsequent processing, in which FIG. 18A shows a case in which the candidate parameter satisfies both the first constraint condition and the second constraint condition, and FIG. 18B shows a case in which the candidate parameter does not satisfy any of the first constraint condition or the second constraint condition.
FIG. 19 is a diagram showing a state in which a candidate parameter from which the most inliers are extracted is adopted as a rigid transformation parameter.
FIG. 20 is a flowchart showing a processing procedure of the apparatus main body.
FIG. 21 is a flowchart showing the processing procedure of the apparatus main body.
FIG. 22 is a flowchart showing the processing procedure of the apparatus main body.
FIG. 23 is a flowchart showing a processing procedure of the apparatus main body in a second embodiment.
FIG. 24 is a flowchart showing the processing procedure of the apparatus main body in the second embodiment.
FIGS. 25A and 25B are diagrams showing an example of changing the constraint condition, in which FIG. 25A shows an example of changing a first distance threshold value to a large value in a case of a technique of causing the ultrasound probe to penetrate, and FIG. 25B shows an example of changing a second distance threshold value to a small value in a case of a technique of obliquely bringing the ultrasound probe into contact.
FIG. 26 is a diagram showing an example of calculating a distance similarity of feature amount vectors of US volume data and transformed CT volume data.

### DESCRIPTION OF THE PREFERRED EMBODIMENTS

### First Embodiment

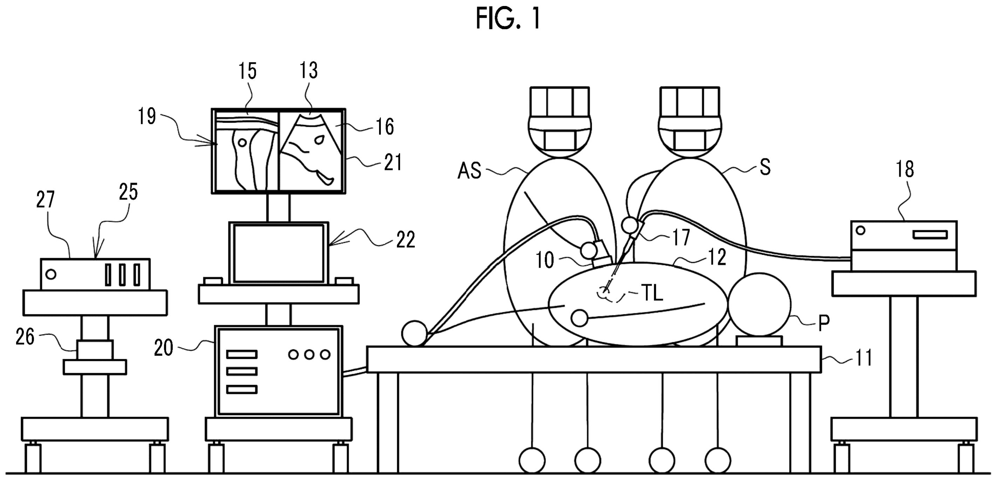

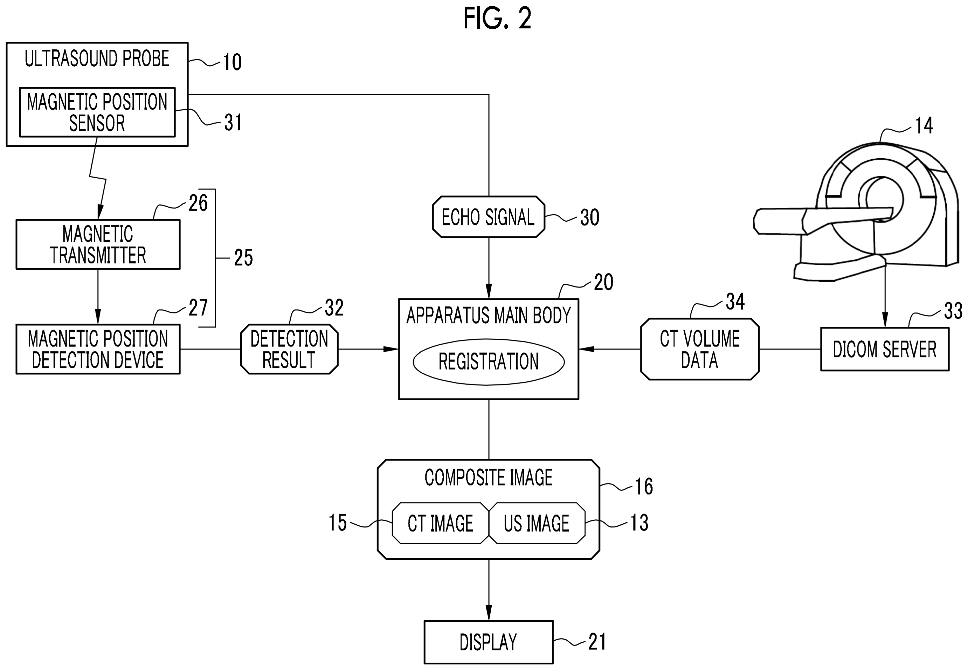

As shown in FIG. 1 as an example, the technology of the present disclosure is a technique of performing display for supporting an operator S by performing registration of an ultrasound image (hereinafter, referred to as an ultrasonography (US) image) 13 captured by bringing an ultrasound probe 10 into contact with a body surface 12 of an abdomen of a patient P lying on a bed 11 by an assistant AS in a perpendicular direction, and a CT image 15 captured by imaging the patient P in a CT apparatus 14 (see FIG. 2) before surgery, in a case in which RFA is performed by a surgical team including the operator S and the assistant AS, and displaying a target site TL of surgery such as a tumor as a marker in a composite image 16 of the US image 13 and the CT image 15. The patient P is an example of a "subject" according to the technology of the present disclosure. The CT image 15 is an example of a "three-dimensional image" according to the technology of the present disclosure.

The operator S makes the distal end of the electrode needle 17 access the target part TL in the body of the patient P by relying on the display of the composite image 16. Then, radio waves are generated from a radio wave generation apparatus 18 to which the electrode needle 17 is connected, and the target site TL is ablated. As described above, the assistant AS brings the ultrasound probe 10 into contact with the body surface 12 of the patient P to search for the target site TL.

The ultrasound probe 10 is connected to an apparatus main body 20. In addition, a display 21 is connected to the apparatus main body 20. A display screen 19 of the composite image 16 in which the US image 13 and the CT image 15 are arranged is displayed on the display 21. An ultrasound diagnostic apparatus 22 is configured by the ultrasound probe 10, the apparatus main body 20, and the display 21. The apparatus main body 20 is an example of an "image processing apparatus" according to the technology of the present disclosure.

In addition to the radio wave generation apparatus 18 and the ultrasound diagnostic apparatus 22, a probe position detection system 25 is installed in an operating room. The probe position detection system 25 is configured by a magnetic transmitter 26 and a magnetic position detection device 27.

As shown in FIG. 2 as an example, the ultrasound probe 10 transmits an echo signal 30 to the apparatus main body 20. In addition, the ultrasound probe 10 incorporates a magnetic position sensor 31. The magnetic position sensor 31 transmits a magnetic position signal to the magnetic transmitter 26. The magnetic transmitter 26 converts the magnetic position signal from the magnetic position sensor 31 into a signal in a format that can be received by the magnetic position detection device 27, and then transmits the signal to the magnetic position detection device 27. The magnetic position detection device 27 detects a position of the ultrasound probe 10 based on the magnetic position signal, and transmits a detection result 32 to the apparatus main body 20.

CT volume data 34 of the patient P is input to the apparatus main body 20 from a digital imaging and communications in medicine (DICOM) server 33 to which the CT apparatus 14 and the like are connected. The CT volume data 34 is generated from a series of CT images 15 of the patient P captured by the CT apparatus 14.

The apparatus main body 20 generates the US image 13 from the echo signal 30. The apparatus main body 20 performs registration of the US image 13 and the CT image 15 based on the detection result 32. Then, the composite image 16 in which the registration result is reflected is generated, and the display screen 19 of the composite image 16 is displayed on the display 21.

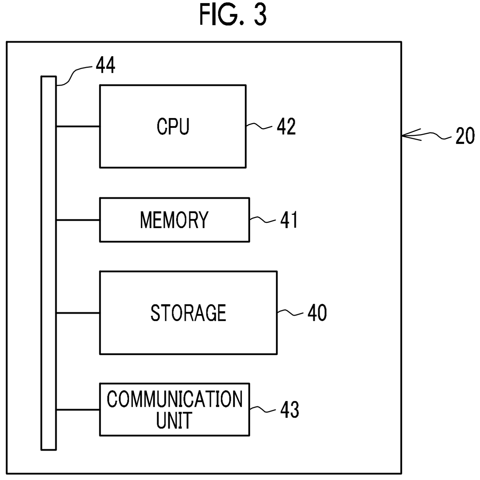

As shown in FIG. 3 as an example, the computer constituting the apparatus main body 20 comprises a storage 40, a memory 41, a central processing unit (CPU) 42, and a communication unit 43. These are interconnected via a busline 44.

The storage 40 is a hard disk drive that is incorporated into the computer that constitutes the apparatus main body 20 or that is connected to the computer through a cable or a network. Alternatively, the storage 40 is a disk array in which a plurality of hard disk drives are connected in series. The storage 40 stores a control program such as an operating system, various application programs, various data associated with these programs, and the like. A solid state drive may be used instead of the hard disk drive.

The memory 41 is a work memory for the CPU 42 to execute processing. The CPU 42 loads the program stored in the storage 40 into the memory 41 and executes processing corresponding to the program. Thus, the CPU 42 integrally controls the respective units of the computer. The CPU 42 is an example of a "processor" according to the technology of the present disclosure. The memory 41 may be built into the CPU 42. The communication unit 43 is a network interface that controls transmission of various types of information to and from an external device.

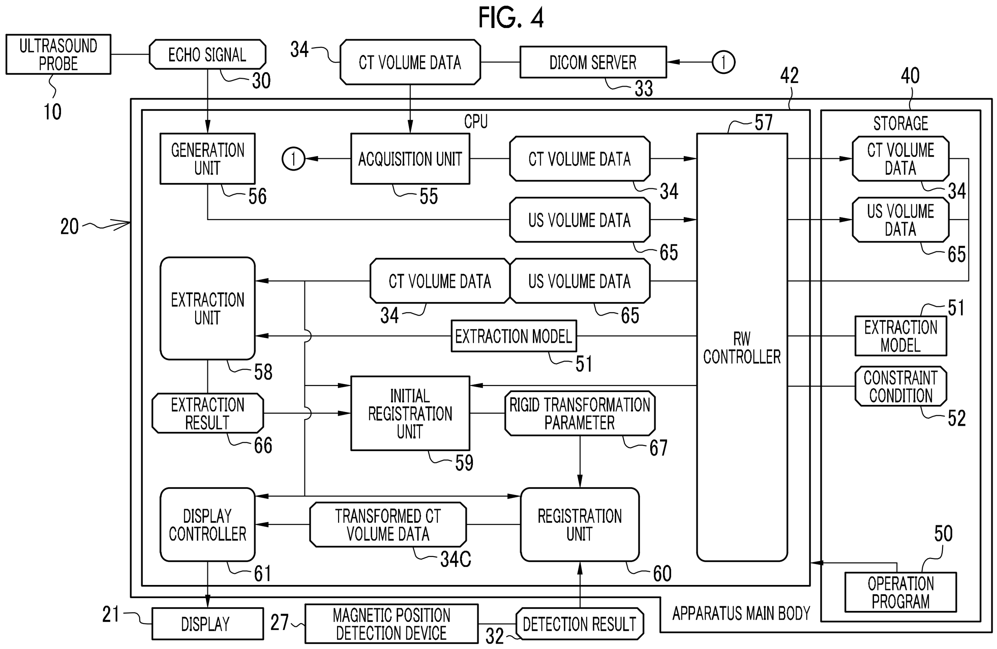

As shown in FIG. 4 as an example, an operation program 50 is stored in the storage 40. The operation program 50 is an application program for causing the computer constituting the apparatus main body 20 to function as the "image processing apparatus" according to the technology of the present disclosure. That is, the operation program 50 is an example of an "operation program of an image processing apparatus" according to the technology of the present disclosure. The storage 40 also stores an extraction model 51 and a constraint condition 52.

In a case in which the operation program 50 is activated, the CPU 42 of the apparatus main body 20 functions as an acquisition unit 55, a generation unit 56, a read/write (hereinafter, referred to as RW) controller 57, an extraction unit 58, an initial registration unit 59, a registration unit 60, and a display controller 61 in cooperation with the memory 41 and the like.

The acquisition unit 55 transmits a transmission request for the CT volume data 34 of the patient P to the DICOM server 33 in response to an instruction from the operator S or the assistant AS. The DICOM server 33 searches for the CT volume data 34 of the patient P corresponding to the transmission request from the database, and transmits the searched CT volume data 34 to the acquisition unit 55. The acquisition unit 55 acquires the CT volume data 34 from the DICOM server 33 by receiving the CT volume data 34. The acquisition unit 55 outputs the CT volume data 34 to the RW controller 57.

The generation unit 56 receives the echo signal 30 from the ultrasound probe 10, and generates the US image 13 based on the echo signal 30. The generation unit 56 generates a plurality of US images 13. Then, US volume data 65 is generated from the plurality of US images 13. The generation unit 56 outputs the US volume data 65 to the RW controller 57.

The RW controller 57 controls the storage of various types of data in the storage 40 and the reading of various types of data from the storage 40. For example, the RW controller 57 stores the CT volume data 34 from the acquisition unit 55 and the US volume data 65 from the generation unit 56 in the storage 40.

The RW controller 57 reads out the CT volume data 34 and the US volume data 65 from the storage 40, and outputs the CT volume data 34 and the US volume data 65 to the extraction unit 58, the initial registration unit 59, the registration unit 60, and the display controller 61. In addition, the RW controller 57 reads out the extraction model 51 from the storage 40, and outputs the extraction model 51 to the extraction unit 58. Further, the RW controller 57 reads out the constraint condition 52 from the storage 40, and outputs the constraint condition 52 to the initial registration unit 59.

The extraction unit 58 extracts a blood vessel and a liver of the liver from the CT volume data 34 and the US volume data 65 using the extraction model 51. Specifically, the blood vessel of the liver is a vein and/or a portal vein of the liver. The extraction unit 58 outputs the extraction result 66 of the blood vessel of the liver and the liver to the initial registration unit 59. The liver is an example of an "organ" according to the technology of the present disclosure.

The initial registration unit 59 performs initial registration as a reference for registration of the US image 13 and the CT image 15 based on the CT volume data 34, the US volume data 65, and the extraction result 66. The initial registration unit 59 outputs a rigid transformation parameter 67 for registration of the US image 13 and the CT image 15 to the registration unit 60 as a result of the initial registration. The rigid transformation parameter 67 is a matrix for performing rigid transformation in which parallel movement and rotation are combined. The rigid transformation parameter 67 is an example of a "transformation parameter" according to the technology of the present disclosure.

The registration unit 60 receives the detection result 32 from the magnetic position detection device 27. The registration unit 60 changes the rigid transformation parameter 67 based on the detection result 32. Then, the CT volume data 34 is transformed using the changed rigid transformation parameter 67, to obtain transformed CT volume data 34C. The registration unit 60 outputs the transformed CT volume data 34C to the display controller 61.

The display controller 61 controls the display of the various screens on the display 21. Various screens include a display screen 19 of the composite image 16 of the US image 13 of the US volume data 65 and the CT image 15 of the transformed CT volume data 34C.

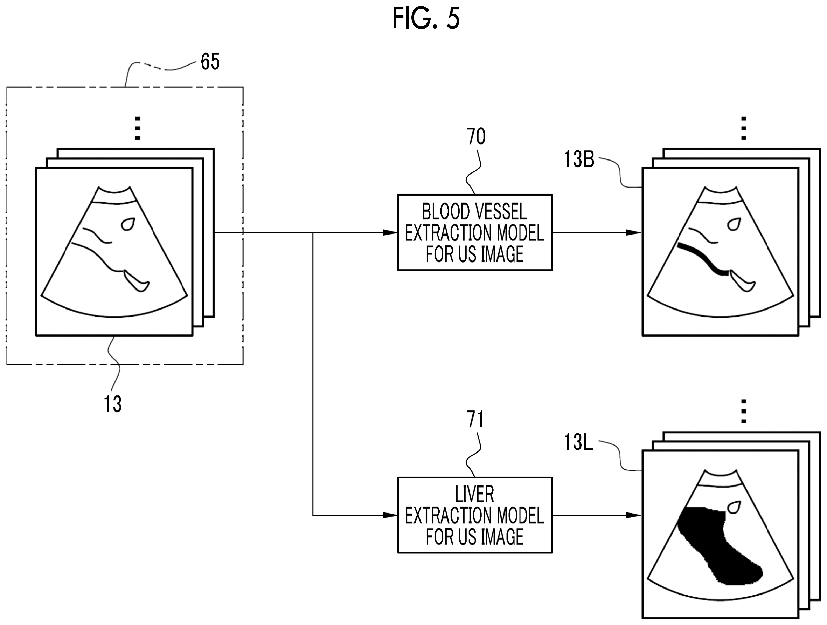

As shown in FIG. 5 as an example, the extraction unit 58 inputs the US volume data 65 to a blood vessel extraction model 70 for a US image and a liver extraction model 71 for a US image. Then, a blood vessel extraction US image 13B is output from the blood vessel extraction model 70 for a US image, and a liver extraction US image 13L is output from the liver extraction model 71 for a US image. The blood vessel extraction US image 13B is an image in which pixels of the US image 13 corresponding to the blood vessel of the liver are labeled. The liver extraction US image 13L is an image in which pixels of the US image 13 corresponding to the liver are labeled.

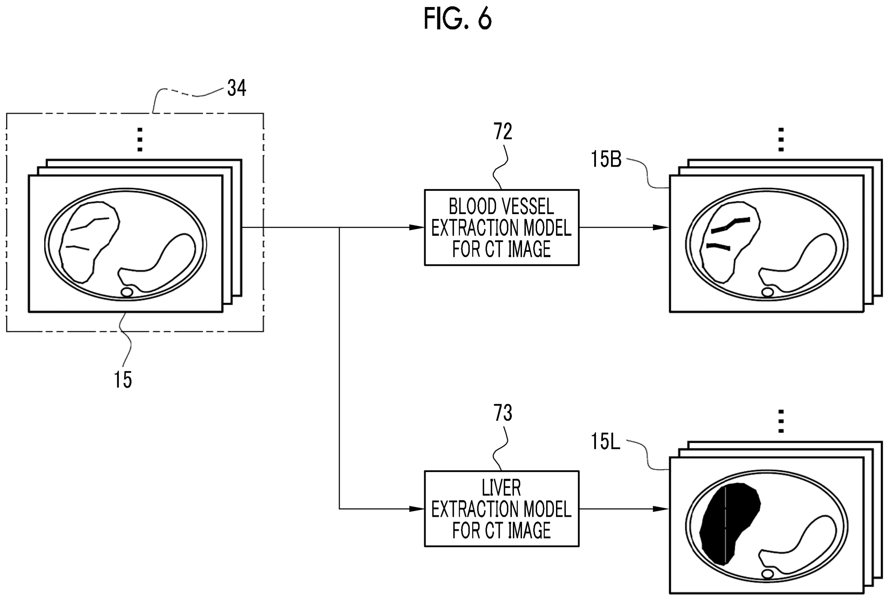

In addition, as shown in FIG. 6 as an example, the extraction unit 58 inputs the CT volume data 34 to a blood vessel extraction model 72 for a CT image and a liver extraction model 73 for a CT image. Then, a blood vessel extraction CT image 15B is output from the blood vessel extraction model 72 for a CT image, and a liver extraction CT image 15L is output from the liver extraction model 73 for a CT image. The blood vessel extraction CT image 15B is an image in which pixels of the CT image 15 corresponding to the blood vessel of the liver are labeled. The liver extraction CT image 15L is an image in which pixels of the CT image 15 corresponding to the liver are labeled.

The blood vessel extraction model 70 for a US image, the liver extraction model 71 for a US image, the blood vessel extraction model 72 for a CT image, and the liver extraction model 73 for a CT image are so-called semantic segmentation models configured by, for example, trained models such as a convolutional neural network (CNN). The extraction model 51 is configured by the blood vessel extraction model 70 for a US image, the liver extraction model 71 for a US image, the blood vessel extraction model 72 for a CT image, and the liver extraction model 73 for a CT image.

In FIG. 5, the US volume data 65 is input to the blood vessel extraction model 70 for a US image and the liver extraction model 71 for a US image, but the US image 13 may be input one by one to the blood vessel extraction model 70 for a US image and the liver extraction model 71 for a US image. Similarly, in FIG. 6, the CT image 15 may be input one by one to the blood vessel extraction model 72 for a CT image and the liver extraction model 73 for a CT image. In addition, the blood vessel extraction model 70 for a US image and the liver extraction model 71 for a US image may be integrated into one extraction model, and the blood vessel extraction US image 13B and the liver extraction US image 13L may be output from the one extraction model. Similarly, the blood vessel extraction model 72 for a CT image and the liver extraction model 73 for a CT image may be integrated into one extraction model, and the blood vessel extraction CT image 15B and the liver extraction CT image 15L may be output from the one extraction model.

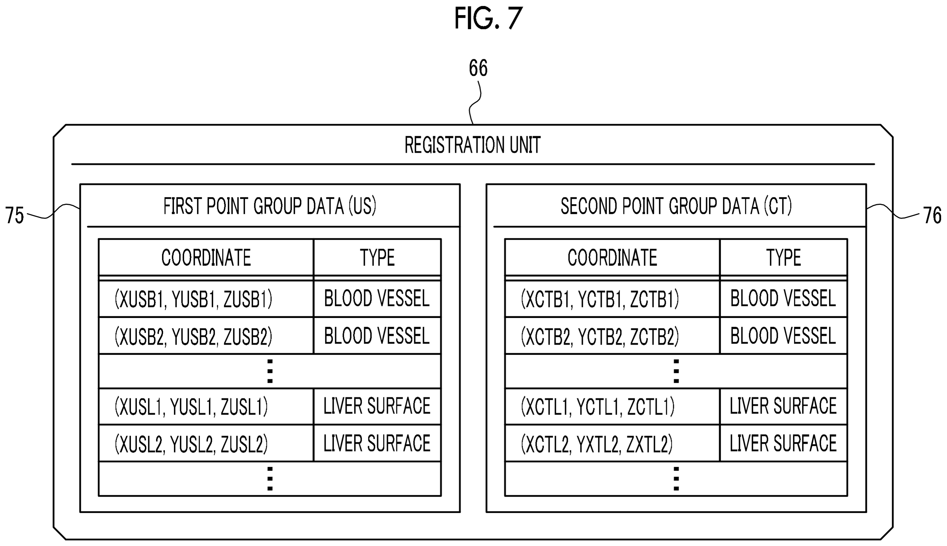

As shown in FIG. 7 as an example, the extraction unit 58 generates first point group data 75 in which three-dimensional coordinates of a pixel (first point) corresponding to the blood vessel labeled in the blood vessel extraction US image 13B and three-dimensional coordinates of a pixel (first point) corresponding to a surface (hereinafter, referred to as a liver surface) of the liver labeled in the liver extraction US image 13L are registered together with a type of the blood vessel or the liver surface. In addition, the extraction unit 58 generates second point group data 76 in which three-dimensional coordinates of a pixel (second point) corresponding to the blood vessel labeled in the blood vessel extraction CT image 15B and three-dimensional coordinates of a pixel (second point) corresponding to the liver surface labeled in the liver extraction CT image 15L are registered together with the type of the blood vessel or the liver surface. As described above, the first point group data 75 is a collection of the pixel corresponding to the blood vessel in the US image 13 and the pixel corresponding to the liver surface. In addition, the second point group data 76 is a collection of the pixel corresponding to the blood vessel in the CT image 15 and the pixel corresponding to the liver surface. The extraction unit 58 outputs a set of the first point group data 75 and the second point group data 76 as the extraction result 66.

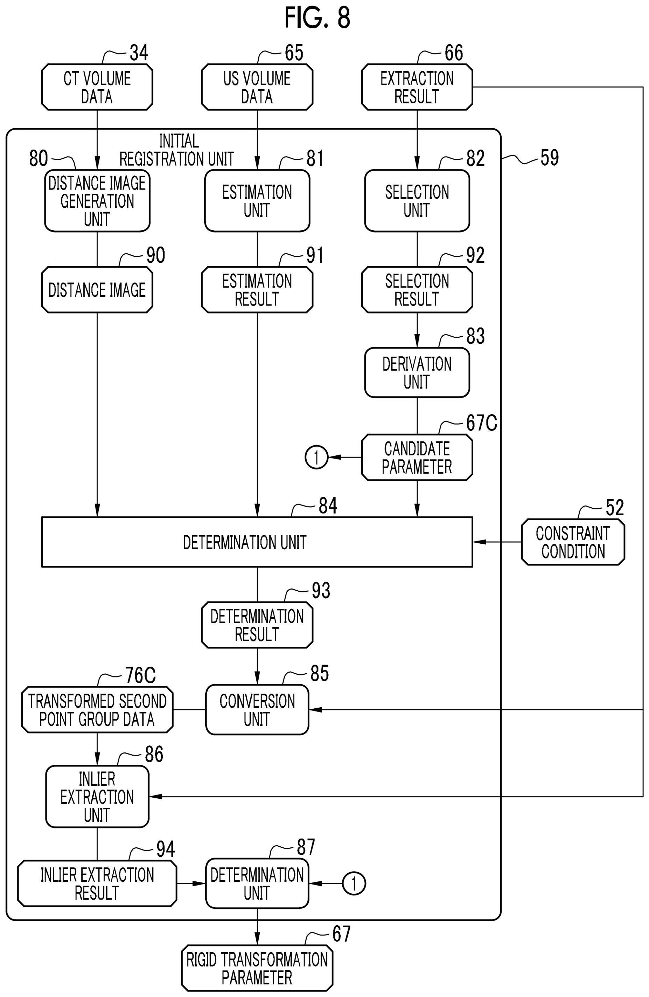

As shown in FIG. 8 as an example, the initial registration unit 59 includes a distance image generation unit 80, an estimation unit 81, a selection unit 82, a derivation unit 83, a determination unit 84, a transformation unit 85, an inlier extraction unit 86, and a decision unit 87.

The distance image generation unit 80 generates a distance image 90 from the CT volume data 34. The distance image generation unit 80 outputs the distance image 90 to the determination unit 84.

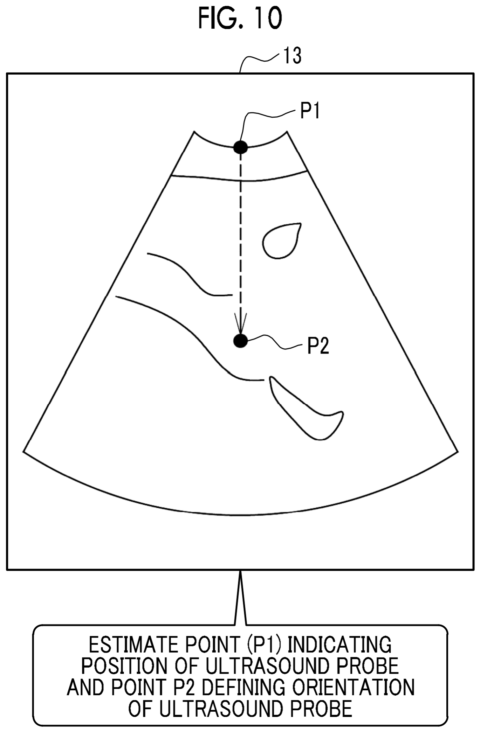

The estimation unit 81 estimates a point P1 (see FIG. 10) indicating a position of the ultrasound probe 10 and a point P2 (see FIG. 10) defining an orientation of the ultrasound probe 10 from one representative US image 13 among the plurality of US images 13 constituting the US volume data 65. The estimation unit 81 outputs an estimation result 91 of the points P1 and P2 to the determination unit 84. The representative one US image 13 is, for example, an image automatically selected by the estimation unit 81. In addition, the representative one US image 13 may be an image designated by the operator S or the assistant AS.

The extraction result 66 is input to the selection unit 82. The selection unit 82 selects the reference point from the first point group data 75 of the extraction result 66 and selects the target point from the second point group data 76. The selection unit 82 outputs a selection result 92 of the reference point and the target point to the derivation unit 83.

The derivation unit 83 derives a candidate (hereinafter, referred to as a candidate parameter) 67C of the rigid transformation parameter 67 from the reference point and the target point of the selection result 92. The derivation unit 83 outputs the candidate parameter 67C to the determination unit 84 and the decision unit 87.

The determination unit 84 determines whether or not the candidate parameter 67C satisfies the constraint condition 52 by using the distance image 90 and the estimation result 91. The determination unit 84 outputs a determination result 93 to the transformation unit 85.

The extraction result 66 is input to the transformation unit 85. The transformation unit 85 transforms the second point group data 76 of the extraction result 66 using the candidate parameter 67C determined by the determination unit 84 to satisfy the constraint condition 52, to obtain transformed second point group data 76C. The transformation unit 85 outputs the transformed second point group data 76C to the inlier extraction unit 86.

The extraction result 66 is input to the inlier extraction unit 86. The inlier extraction unit 86 extracts an inlier from the first point group based on the first point group data 75 of the extraction result 66 and the transformed second point group data 76C from the transformation unit 85. Since a method of extracting the inlier is based on a well-known algorithm of random sample consensus (RANSAC), detailed description thereof will be omitted. In brief, the inlier is extracted as the first point at which a distance from the second point of the transformed second point group data 76C is small and an estimated traveling direction of the blood vessel or an estimated normal direction of the liver surface matches the direction of the second point of the transformed second point group data 76C, and the outlier is extracted as the first point other than the first point. The inlier extraction unit 86 outputs an inlier extraction result 94 to the decision unit 87. The inlier extraction result 94 is an example of a "similarity" according to the technology of the present disclosure. Therefore, extracting the inlier is an example of "calculating the similarity" according to the technology of the present disclosure. In addition, the inlier is an example of a "point that is not an outlier" according to the technology of the present disclosure. The outlier is an example of an "outlier" according to the technology of the present disclosure.

The selection unit 82 repeats the selection of the reference point and the target point a plurality of times, for example, a predetermined number of times (hereinafter, referred to as a set number of times). The derivation unit 83 derives the candidate parameter 67C each time the selection unit 82 selects the reference point and the target point. The determination unit 84 determines whether or not the candidate parameter 67C satisfies the constraint condition 52 each time the derivation unit 83 derives the candidate parameter 67C. Therefore, a plurality of candidate parameters 67C are input to the decision unit 87.

The decision unit 87 stores the plurality of candidate parameters 67C from the derivation unit 83 and the inlier extraction result 94 from the inlier extraction unit 86. The decision unit 87 determines the rigid transformation parameter 67 to be adopted from among the plurality of candidate parameters 67C based on the inlier extraction result 94.

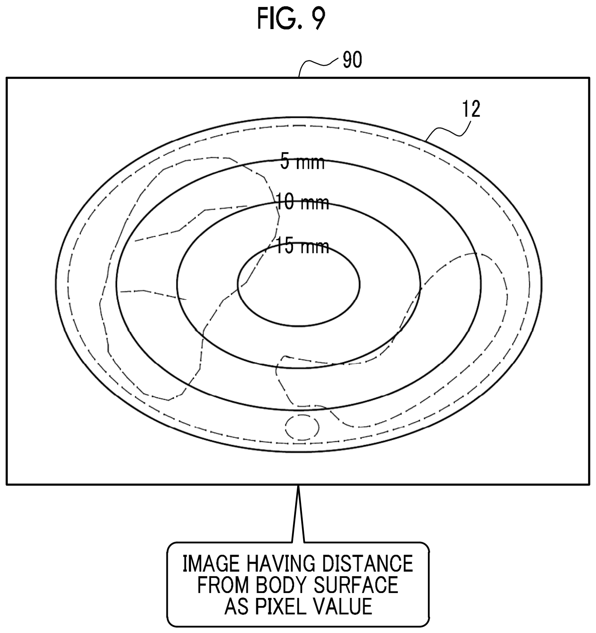

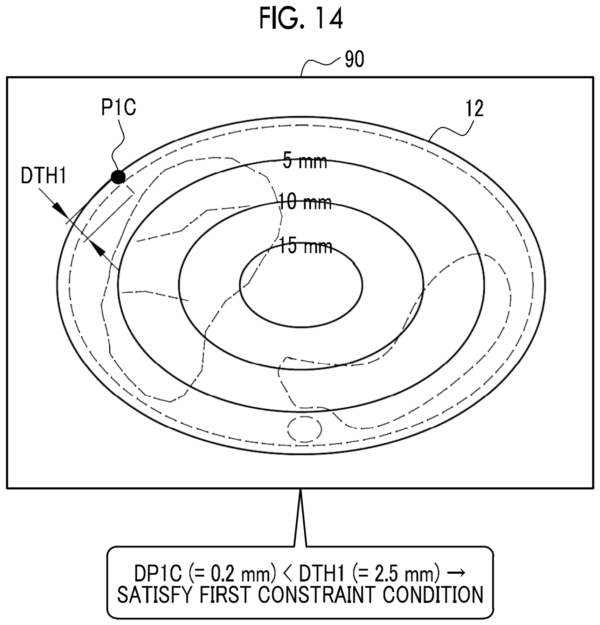

As shown in FIG. 9 as an example, the distance image 90 is an image in which a pixel representing each site in the body of the patient P shown in the CT image 15 is registered with a distance from the body surface 12 as a pixel value. In FIG. 9, for convenience, a line indicating that the distance from the body surface 12 is 5 mm, 10 mm, and 15 mm is drawn, but the actual distance image 90 does not have such a line. The distance image 90 shown in FIG. 9 is one of a plurality of pieces of distance image 90 constituting the volume data of the distance image 90 generated from the CT volume data 34. The same applies to FIGS. 14 to 17.

As shown in FIG. 10 as an example, the estimation unit 81 estimates a center point of an upper end of a field of view (FOV) of the US image 13 as the point P1 indicating the position of the ultrasound probe 10. In addition, the estimation unit 81 estimates a center point of the FOV of the US image 13 and a point through which a vector from the point P1 in an irradiation direction of the ultrasound is a point P2 that defines the orientation of the ultrasound probe 10.

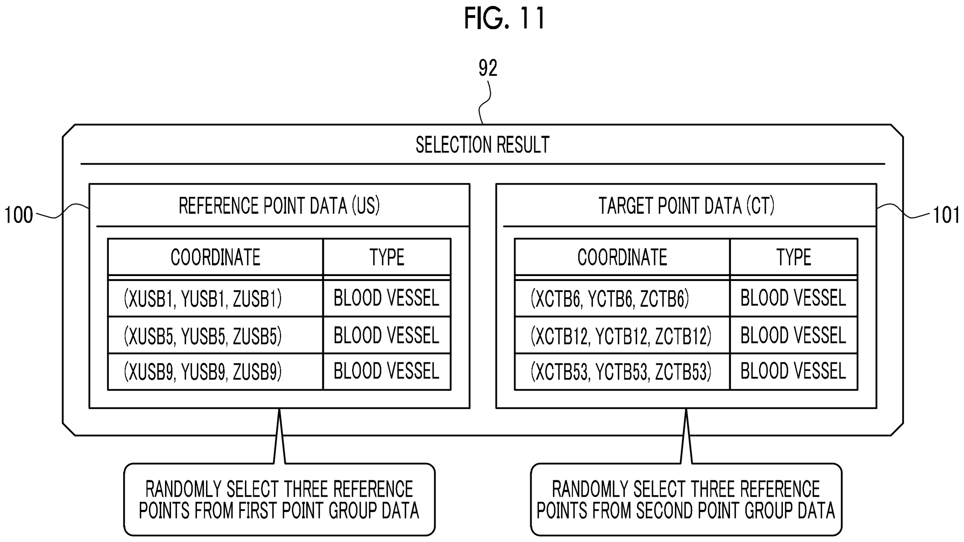

As shown in FIG. 11 as an example, the selection unit 82 randomly extracts three reference points from a plurality of first points of the same type in the first point group data 75 of the extraction result 66. Then, reference point data 100 in which the three-dimensional coordinates of the extracted reference point are registered is generated. Similarly, the selection unit 82 randomly extracts three target points from a plurality of second points of the same type in the second point group data 76 of the extraction result 66. Then, target point data 101 in which the three-dimensional coordinates of the extracted target point are registered is generated. The selection unit 82 outputs a set of the reference point data 100 and the target point data 101 as the selection result 92. FIG. 11 illustrates a case in which the first point and the second point of the type of the blood vessel are selected as the reference point and the target point.

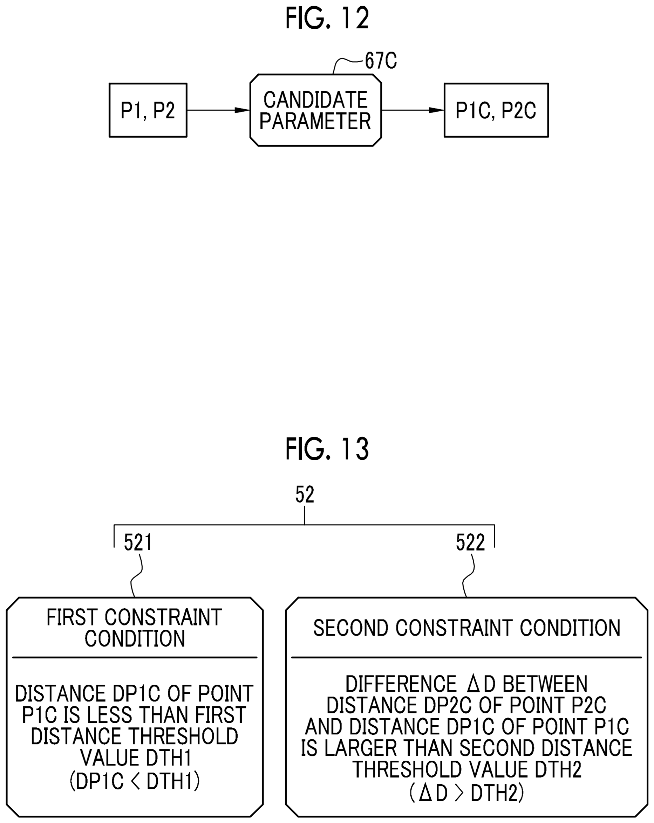

As shown in FIG. 12 as an example, the determination unit 84 transforms the points P1 and P2 of the estimation result 91 using the candidate parameter 67C, to obtain points P1C and P2C. By transforming the points P1 and P2 into the points P1C and P2C in this way, it is possible to determine whether or not the candidate parameter 67C satisfies the constraint condition 52 by using the distance image 90 generated from the CT image 15.

As shown in FIG. 13 as an example, the constraint condition 52 is composed of a first constraint condition 521 and a second constraint condition 522. The first constraint condition 521 has a content that a distance DP1C of the point P1C from the body surface 12 is less than a first distance threshold value DTH1 related to the distance from the body surface 12 (DP1C < DTH1). That is, the first constraint condition 521 is a constraint condition related to the position of the ultrasound probe 10. More specifically, the first constraint condition 521 is a condition related to the distance between the position of the ultrasound probe 10 and the body surface 12.

The second constraint condition 522 has a content that a difference ΔD between a distance DP2C of the point P2C from the body surface 12 and a distance DP1C of the point P1C from the body surface 12 is larger than a second distance threshold value DTH2 related to the orientation of the ultrasound probe 10 with respect to the body surface 12 (ΔD > DTH2). That is, the second constraint condition 522 is a constraint condition related to the posture of the ultrasound probe 10. More specifically, the second constraint condition 522 is a condition related to the orientation of the ultrasound probe 10 with respect to the body surface 12.

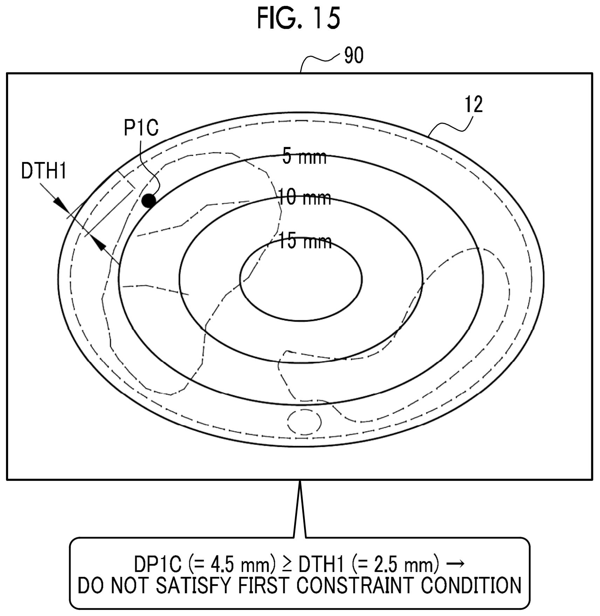

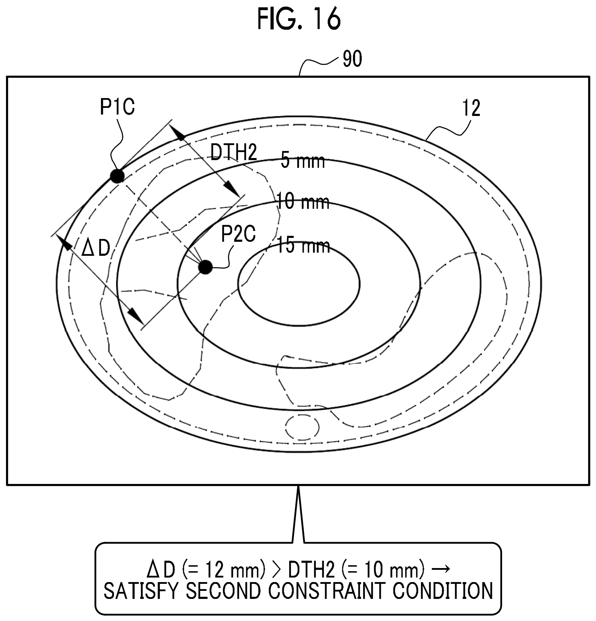

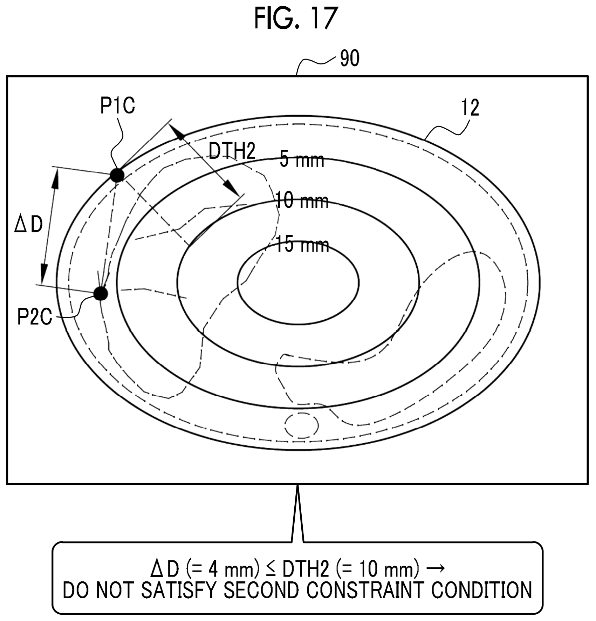

FIGS. 14 and 15 show a state in which it is determined whether or not the candidate parameter 67C satisfies the first constraint condition 521 by using the distance image 90. In addition, FIGS. 16 and 17 show a state in which it is determined whether or not the candidate parameter 67C satisfies the second constraint condition 522 by using the distance image 90.

FIG. 14 shows a case in which the distance DP1C of the point P1C from the body surface 12 is 0.2 mm and is less than 2.5 mm of the first distance threshold value DTH1. In this case, the determination unit 84 determines that the candidate parameter 67C satisfies the first constraint condition 521. On the other hand, FIG. 15 shows a case in which the distance DP1C of the point P1C from the body surface 12 is 4.5 mm and is 2.5 mm or more of the first distance threshold value DTH1. In this case, the determination unit 84 determines that the candidate parameter 67C does not satisfy the first constraint condition 521. The 2.5 mm of the first distance threshold value DTH1 is merely an example.

As can be seen from the description of FIGS. 14 and 15, the first constraint condition 521 is a condition set on the premise that the ultrasound probe 10 is brought into contact with the body surface 12 and the position of the ultrasound probe 10 is not present in the body of the patient P.

FIG. 16 shows a case in which the difference ΔD between the distance DP2C of the point P2C from the body surface 12 and the distance DP1C of the point P1C from the body surface 12 is 12 mm and is larger than 10 mm of the second distance threshold value DTH2. In this case, the determination unit 84 determines that the candidate parameter 67C satisfies the second constraint condition 522. On the other hand, FIG. 17 shows a case in which the difference ΔD between the distance DP2C of the point P2C from the body surface 12 and the distance DP1C of the point P1C from the body surface 12 is 4 mm and is 10 mm or less of the second distance threshold value DTH2. In this case, the determination unit 84 determines that the candidate parameter 67C does not satisfy the second constraint condition 522. The 10 mm of the second distance threshold value DTH2 is also merely an example, as in the first distance threshold value DTH1.

As can be seen from the description of FIGS. 16 and 17, the second constraint condition 522 is a condition set on the premise that the ultrasound probe 10 is brought into contact with the body surface 12 toward the body and the posture of the ultrasound probe 10 does not deviate from a direction toward the body from the body surface 12. Furthermore, the second constraint condition 522 is a condition for determining whether or not a vector connecting the point P1C and the point P2C is a normal line of the body surface 12 and a direction of the vector is toward the body from the body surface 12.

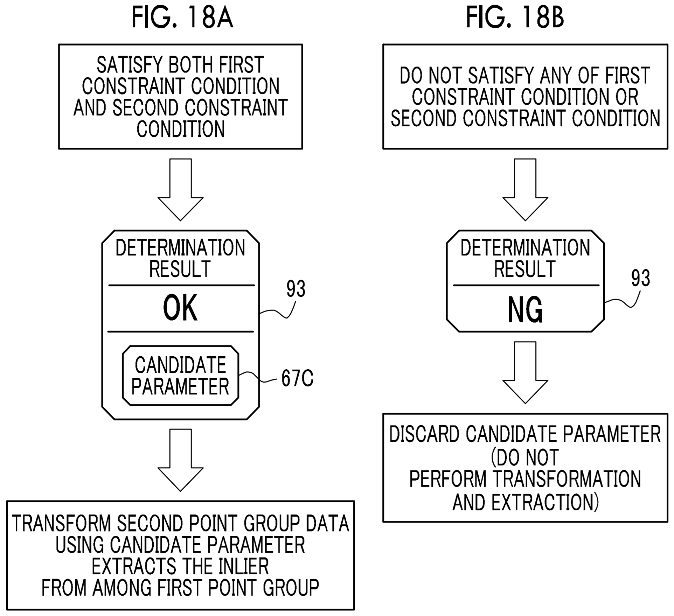

As shown in FIG. 18A as an example, in a case in which the determination unit 84 determines that the candidate parameter 67C satisfies both the first constraint condition 521 and the second constraint condition 522, the determination unit 84 outputs the determination result 93 indicating that the candidate parameter 67C satisfies the constraint condition 52. The determination unit 84 includes the candidate parameter 67C in the determination result 93. In this case, the transformation unit 85 transforms the second point group data 76 using the candidate parameter 67C, and the inlier extraction unit 86 extracts the inlier from the first point group.

On the other hand, as shown in FIG. 18B as an example, in a case in which the determination unit 84 determines that the candidate parameter 67C does not satisfy any of the first constraint condition 521 or the second constraint condition 522, the determination unit 84 outputs the determination result 93 indicating that the candidate parameter 67C does not satisfy the constraint condition 52. In this case, the determination unit 84 does not include the candidate parameter 67C in the determination result 93, and discards the candidate parameter 67C. Therefore, in this case, the transformation unit 85 does not transform the second point group data 76, and the inlier extraction unit 86 does not extract the inlier.

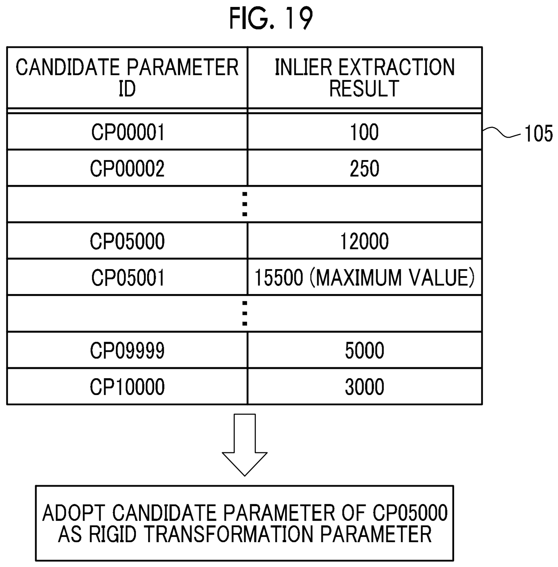

As shown in a table 105 in FIG. 19 as an example, the decision unit 87 stores the inlier extraction result 94 of each of the plurality of candidate parameters 67C. In a case in which the selection unit 82 ends the selection of the number of times of setting the reference point and the target point, and the derivation unit 83 ends the derivation of the last candidate parameter 67C, the decision unit 87 compares the inlier extraction results 94 of the candidate parameters 67C. Then, the candidate parameter 67C having the most inlier extraction results 94 is determined to be adopted as the rigid transformation parameter 67. FIG. 19 illustrates a case in which the inlier extraction result 94 of the candidate parameter 67C having a candidate parameter identification data (ID) of "CP05001" is the maximum of "15500", and the candidate parameter 67C of "CP05001" is adopted as the rigid transformation parameter 67. The candidate parameter 67C having the most inlier extraction results 94 is an example of a "candidate having the highest similarity" according to the technology of the present disclosure.

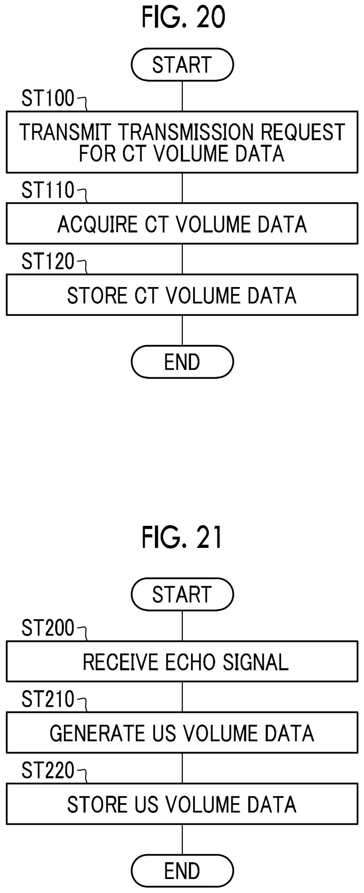

Next, the operation of the above configuration will be described with reference to the flowcharts shown in FIGS. 20, 21, and 22 as an example. As shown in FIG. 4, the CPU 42 of the apparatus main body 20 functions as the acquisition unit 55, the generation unit 56, the RW controller 57, the extraction unit 58, the initial registration unit 59, the registration unit 60, and the display controller 61 by activating the operation program 50.

A transmission request for the CT volume data 34 of the patient P is transmitted from the acquisition unit 55 to the DICOM server 33 in response to an instruction from the operator S or the assistant AS (step ST100 in FIG. 20). Then, the CT volume data 34 of the patient P transmitted from the DICOM server 33 in response to the transmission request is acquired by the acquisition unit 55 (step ST110). The CT volume data 34 is output from the acquisition unit 55 to the RW controller 57, and is stored in the storage 40 by the RW controller 57 (step ST120).

The assistant AS scans the body surface 12 of the patient P with the ultrasound probe 10, and the echo signal 30 is captured by the ultrasound probe 10. The echo signal 30 from the ultrasound probe 10 is received by the generation unit 56 (step ST200 in FIG. 21). The generation unit 56 generates the US image 13 based on the echo signal 30, and generates the US volume data 65 from the plurality of US images 13 (step ST210). The US volume data 65 is output from the generation unit 56 to the RW controller 57, and is stored in the storage 40 by the RW controller 57 (step ST220).

The CT volume data 34 and the US volume data 65 are read out from the storage 40 by the RW controller 57, and are output from the RW controller 57 to the extraction unit 58, the initial registration unit 59, the registration unit 60, and the display controller 61. In addition, the extraction model 51 is read out from the storage 40 by the RW controller 57, and is output to the extraction unit 58. Further, the constraint condition 52 is read out from the storage 40 by the RW controller 57, and is output to the initial registration unit 59.

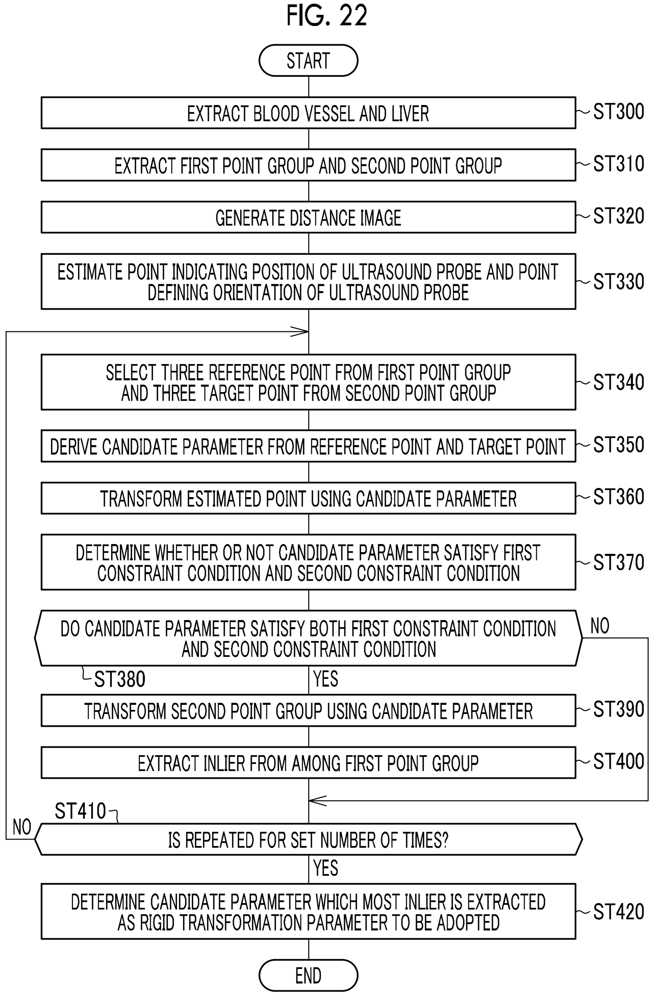

As shown in FIGS. 5 and 6, in the extraction unit 58, the blood vessel extraction US image 13B and the liver extraction US image 13L are output from the US volume data 65, and the blood vessel extraction CT image 15B and the liver extraction CT image 15L are output from the CT volume data 34 by using the blood vessel extraction model 70 for a US image, the liver extraction model 71 for a US image, the blood vessel extraction model 72 for a CT image, and the liver extraction model 73 for a CT image, which are the extraction model 51 (step ST300 in FIG. 22). Then, as shown in FIG. 7, the extraction unit 58 generates the extraction result 66 composed of the set of the first point group data 75 and the second point group data 76 (step ST310). The first point group data 75 is data in which the three-dimensional coordinates of the pixel corresponding to the blood vessel labeled in the blood vessel extraction US image 13B and the three-dimensional coordinates of the pixel corresponding to the liver surface labeled in the liver extraction US image 13L are registered. The second point group data 76 is data in which the three-dimensional coordinates of the pixel corresponding to the blood vessel labeled in the blood vessel extraction CT image 15B and the three-dimensional coordinates of the pixel corresponding to the liver surface labeled in the liver extraction CT image 15L are registered. The extraction result 66 is output from the extraction unit 58 to the initial registration unit 59.

As shown in FIG. 8, in the initial registration unit 59, the CT volume data 34 is input to the distance image generation unit 80, and the US volume data 65 is input to the estimation unit 81. In addition, the extraction result 66 is input to the selection unit 82, the transformation unit 85, and the inlier extraction unit 86.

The distance image generation unit 80 generates the distance image 90 shown in FIG. 9 from the CT volume data 34 (step ST320). The distance image 90 is output from the distance image generation unit 80 to the determination unit 84.

In addition, as shown in FIG. 10, the estimation unit 81 estimates the point P1 indicating the position of the ultrasound probe 10 and the point P2 defining the orientation of the ultrasound probe 10 (step ST330). The estimation result 91 of the points P1 and P2 is output from the estimation unit 81 to the determination unit 84.

As shown in FIG. 11, the selection unit 82 randomly selects three reference points from the first point group data 75 and randomly selects three target points from the second point group data 76 (step ST340). The selection result 92 of the reference point and the target point is output from the selection unit 82 to the derivation unit 83.

In the derivation unit 83, the candidate parameter 67C is derived from the reference point and the target point of the selection result 92 (step ST350). The candidate parameter 67C is output from the derivation unit 83 to the determination unit 84 and the decision unit 87.

As shown in FIG. 12, the points P1 and P2 in the estimation result 91 are transformed into the points P1C and P2C using the candidate parameter 67C by the determination unit 84 (step ST360). Then, as shown in FIGS. 14 to 17, it is determined whether or not the candidate parameter 67C satisfies the first constraint condition 521 and the second constraint condition 522 based on the distance image 90 and the points P1C and P2C (step ST370).

In a case in which the candidate parameter 67C satisfies both the first constraint condition 521 and the second constraint condition 522 (YES in step ST380), as shown in FIG. 18A, the determination result 93 in which the candidate parameter 67C satisfies the constraint condition 52 and the candidate parameter 67C is included is output from the determination unit 84 to the transformation unit 85. In this case, in the transformation unit 85, the second point group data 76 is transformed using the candidate parameter 67C, to obtain transformed second point group data 76C (step ST390). The transformed second point group data 76C is output from the transformation unit 85 to the inlier extraction unit 86.

In the inlier extraction unit 86, the inlier is extracted from the first point group (step ST400). The inlier extraction result 94 is output from the inlier extraction unit 86 to the decision unit 87. A series of processes of steps ST340 to ST400 are repeatedly performed until the selection of the number of times of setting the reference point and the target point by the selection unit 82 is ended (NO in step ST410).

In a case in which the selection of the number of times of setting the reference point and the target point by the selection unit 82 is ended (YES in step ST410), as shown in FIG. 19, in the decision unit 87, the candidate parameter 67C having the most inlier extraction results 94 among the plurality of candidate parameters 67C is determined as the rigid transformation parameter to be adopted (step ST420). The rigid transformation parameter 67 is output from the initial registration unit 59 to the registration unit 60. As a result, the initial registration by the initial registration unit 59 is ended.

After the initial registration is ended, the magnetic position detection device 27 operates, and the detection result 32 of the position of the ultrasound probe 10 is input to the registration unit 60. In the registration unit 60, the rigid transformation parameter 67 is changed based on the detection result 32, and the CT volume data 34 is transformed using the changed rigid transformation parameter 67, to obtain transformed CT volume data 34C. The transformed CT volume data 34C is output from the registration unit 60 to the display controller 61.

In the display controller 61, the display screen 19 of the composite image 16 of the US image 13 of the US volume data 65 and the CT image 15 of the transformed CT volume data 34 is generated. The display screen 19 is displayed on the display 21 under the control of the display controller 61.

As described above, the CPU 42 of the apparatus main body 20 functions as the acquisition unit 55, the generation unit 56, and the initial registration unit 59. The initial registration unit 59 includes the selection unit 82, the derivation unit 83, and the determination unit 84. The acquisition unit 55 acquires the CT image 15 of the patient P. The generation unit 56 acquires the US image 13 of the patient P by generating the US image 13 from the echo signal 30. The selection unit 82 repeatedly selects a plurality of reference points from the first point group of the US image 13 and a plurality of target points from the second point group of the CT image 15 a plurality of times. The derivation unit 83 derives the candidate parameter 67C each time the reference point and the target point are selected. The determination unit 84 determines whether or not the candidate parameter 67C satisfies the constraint condition 52 related to the position and the posture of the ultrasound probe 10 at the time of capturing the US image 13.

Since the selection unit 82 repeatedly selects the reference point and the target point a plurality of times, and the derivation unit 83 derives the candidate parameter 67C each time the reference point and the target point are selected, various candidate parameters 67C can be obtained. Therefore, as in the ICP algorithm used in the technique described in US2014/0193053A, the rigid transformation parameter 67 is not likely to fall into a local solution due to the initial setting of the reference point and the target point.

Since the determination unit 84 determines whether or not the candidate parameter 67C satisfies the constraint condition 52, it is possible to suppress the output of a clearly incorrect rigid transformation parameter 67. As a result, the rigid transformation parameter 67 with high relative positioning accuracy can be adopted.

The transformation unit 85 transforms the second point group using the candidate parameter 67C determined to satisfy the constraint condition 52. The inlier extraction unit 86 extracts the inlier from the first point group based on the first point group and the transformed second point group. On the other hand, the transformation unit 85 and the inlier extraction unit 86 do not perform the transformation and the extraction of the inlier on the candidate parameter 67C determined not to satisfy the constraint condition 52. Therefore, it is possible to reduce the processing load and shorten the processing time as compared with a case in which the transformation and the extraction of the inlier are performed on all the candidate parameters 67C. In particular, in the technology of the present disclosure in which the selection unit 82 repeatedly selects the reference point and the target point a plurality of times and the derivation unit 83 derives the candidate parameter 67C each time the reference point and the target point are selected, the number of candidate parameters 67C, and thus the number of times of the transformation and the inlier extraction, is extremely large. Therefore, the effect of reducing the processing load and shortening the processing time is very meaningful.

As shown in FIG. 19, the decision unit 87 adopts the candidate parameter 67C from among the plurality of candidate parameters 67C determined to satisfy the constraint condition 52, from which the most inliers are extracted, as the rigid transformation parameter 67. According to the candidate parameter 67C from which the most inliers are extracted, the similarity between the US image 13 and the transformed CT image 15 can be maximized. That is, the registration accuracy between the US image 13 and the CT image 15 can be further improved.

As shown in FIGS. 5 to 7, the first point group and the second point group are point groups related to the liver and the blood vessel shown in the US image 13 and the CT image 15. The liver and the blood vessel thereof are easily extracted in any of the US image 13 and the CT image 15. Therefore, the first point group and the second point group that are necessary and sufficient for the registration can be extracted. The first point group and the second point group may be point groups related to any one of the liver or the blood vessel. In addition, the organ is not limited to the liver as an example, and may be another organ such as a kidney and a spleen.

As shown in FIGS. 14 to 17, the determination unit 84 determines whether or not the candidate parameter 67C satisfies the first constraint condition 521 related to the position of the ultrasound probe 10, and determines whether or not the candidate parameter 67C satisfies the second constraint condition 522 related to the posture of the ultrasound probe 10. Therefore, the effect of suppressing the output of the clearly incorrect rigid transformation parameter 67 can be further enhanced.

It should be noted that the determination of whether or not the candidate parameter 67C satisfies the first constraint condition 521 may be limited, and the determination of whether or not the candidate parameter 67C satisfies the second constraint condition 522 may not be performed. On the contrary, the determination of whether or not the candidate parameter 67C satisfies the second constraint condition 522 may be limited, and the determination of whether or not the candidate parameter 67C satisfies the first constraint condition 521 may not be performed. However, in these cases, the effect of suppressing the output of the clearly incorrect rigid transformation parameter 67 is reduced.

As shown in FIG. 13, the first constraint condition 521 is a condition related to the distance between the position of the ultrasound probe 10 and the distance and the body surface 12 of the patient P in the CT image 15. Therefore, the candidate parameter 67C that is clearly incorrect, such as the position of the ultrasound probe 10 being inside the body of the patient P, can be easily discarded.

As shown in FIG. 13, the second constraint condition 522 is a condition related to the orientation of the ultrasound probe 10 with respect to the body surface 12 of the patient P in the CT image 15. Therefore, the candidate parameter 67C that is clearly incorrect, such as the orientation of the ultrasound probe 10 being oblique with respect to the body surface 12, can be easily discarded.

As shown in FIG. 9, the distance image generation unit 80 generates the distance image 90 representing the distance from the body surface 12 of the patient P from the CT image 15. As shown in FIGS. 14 to 17, the determination unit 84 determines whether or not the candidate parameter 67C satisfies the constraint condition 52 by using the distance image 90. Therefore, it is possible to simply and accurately determine whether or not the candidate parameter 67C satisfies the constraint condition 52.

### Second Embodiment

In the first embodiment, it is not defined which of the first point group and the second point group of the type of the blood vessel and the first point group and the second point group of the type of the liver surface is prioritized, but the present disclosure is not limited thereto.

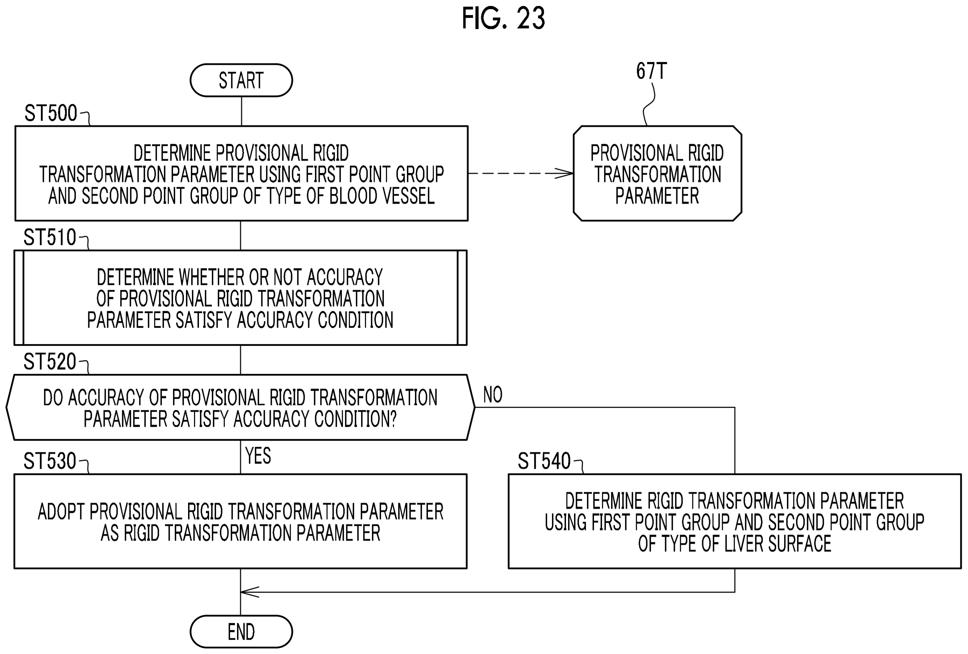

In the second embodiment, the first point group and the second point group of the type of the blood vessel are prioritized. That is, as shown in a flowchart in FIG. 23 as an example, the initial registration unit 59 first determines a provisional rigid transformation parameter 67T using the first point group and the second point group of the type of the blood vessel (step ST500). More specifically, in step ST340 of the flowchart shown in FIG. 22, the selection unit 82 selects three reference points from the first point group of the type of the blood vessel and selects three target points from the second point group of the type of the blood vessel. Then, after the processes of steps ST340 to ST400 are repeated for the set number of times, the process of step ST420 is executed to determine the provisional rigid transformation parameter 67T.

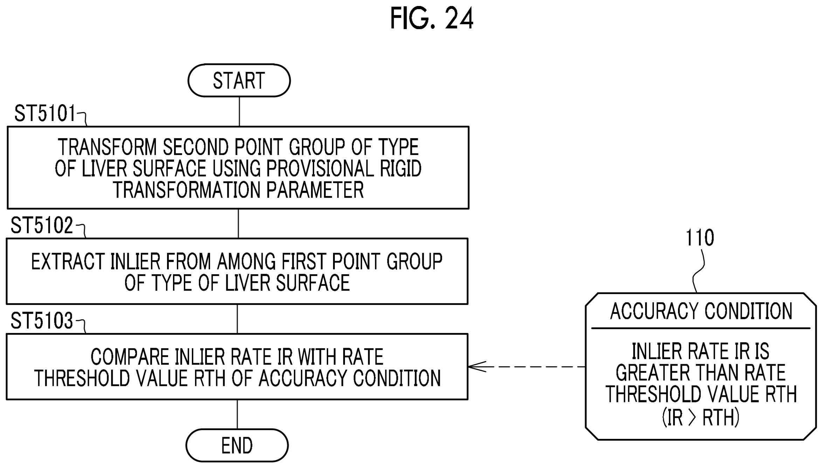

The initial registration unit 59 determines whether or not the accuracy of the provisional rigid transformation parameter 67T satisfies an accuracy condition 110 (see FIG. 24) (step ST510). Here, the accuracy of the provisional rigid transformation parameter 67T is the registration accuracy between the US image 13 and the CT image 15. The provisional rigid transformation parameter 67T that can successfully perform the registration of the US image 13 and the CT image 15 has high accuracy.

In a case in which it is determined that the accuracy of the provisional rigid transformation parameter 67T satisfies the accuracy condition 110 (YES in step ST520), the initial registration unit 59 adopts the provisional rigid transformation parameter 67T as the rigid transformation parameter 67 (step ST530). On the other hand, in a case in which it is determined that the accuracy of the provisional rigid transformation parameter 67T does not satisfy the accuracy condition 110 (NO in step ST520), the initial registration unit 59 determines the rigid transformation parameter 67 using the first point group and the second point group of the type of the liver surface (step ST540). More specifically, in step ST340 of the flowchart shown in FIG. 22, the selection unit 82 selects three reference points from the first point group of the type of the liver surface and selects three target points from the second point group of the type of the liver surface. Then, after the processes of steps ST340 to ST400 are repeated for the set number of times, the process of step ST420 is executed to determine the rigid transformation parameter 67.

Details of the determination in step ST510 are, for example, as shown in FIG. 24, in which the initial registration unit 59 transforms the second point group of the type of the liver surface using the provisional rigid transformation parameter 67T (step ST5101). Next, the initial registration unit 59 extracts the inlier from the first point group of the type of the liver surface based on the transformed second point group (step ST5102).

The initial registration unit 59 calculates an inlier rate IR by dividing the number of extracted inliers by the total number of first points constituting the first point group of the type of the liver surface. The initial registration unit 59 compares the inlier rate IR with a rate threshold value RTH of the accuracy condition 110 (step ST5103). The accuracy condition 110 has a content that the inlier rate IR is greater than the rate threshold value RTH (IR > RTH). The accuracy condition 110 is stored in the storage 40, and is read out from the storage 40 by the RW controller 57 and is input to the initial registration unit 59.

As described above, in the second embodiment, the initial registration unit 59 determines the provisional rigid transformation parameter 67T using the first point group and the second point group of the type of the blood vessel, and determines whether or not the accuracy of the provisional rigid transformation parameter 67T satisfies the accuracy condition 110. In a case in which it is determined that the accuracy of the provisional rigid transformation parameter 67T satisfies the accuracy condition 110, the initial registration unit 59 adopts the provisional rigid transformation parameter 67T as the rigid transformation parameter 67. On the other hand, in a case in which it is determined that the accuracy of the provisional rigid transformation parameter 67T does not satisfy the accuracy condition 110, the initial registration unit 59 determines the rigid transformation parameter 67 using the first point group and the second point group of the type of the liver surface.

By using the first point group and the second point group of the type of the blood vessel, the rigid transformation parameter 67 having higher accuracy can be obtained as compared with a case in which the first point group and the second point group of the type of the liver surface are used. Therefore, in a case in which the first point group and the second point group of the type of the blood vessel are prioritized, the possibility of adopting the rigid transformation parameter 67 having relatively high accuracy is increased. In addition, in a case in which the accuracy of the provisional rigid transformation parameter 67T satisfies the accuracy condition 110, the processing using the first point group and the second point group of the type of the liver surface does not need to be executed, so that it is possible to contribute to further reduction of the processing load and shortening of the processing time.

In a case in which the provisional rigid transformation parameter 67T obtained by using the first point group and the second point group of the type of the blood vessel is adopted as the rigid transformation parameter 67 without checking the accuracy of the provisional rigid transformation parameter 67T, and the accuracy of the provisional rigid transformation parameter 67T is low, the registration is hindered. In addition, there is also a patient P in whom the blood vessel is difficult to see due to the body type. Therefore, the initial registration unit 59 determines whether or not the accuracy of the provisional rigid transformation parameter 67T satisfies the accuracy condition 110. Therefore, it is possible to prevent the provisional rigid transformation parameter 67T having low accuracy from being adopted as the rigid transformation parameter 67. In addition, the rigid transformation parameter 67 can be obtained without any problem even for the patient P in whom the blood vessel is difficult to see due to the body type.

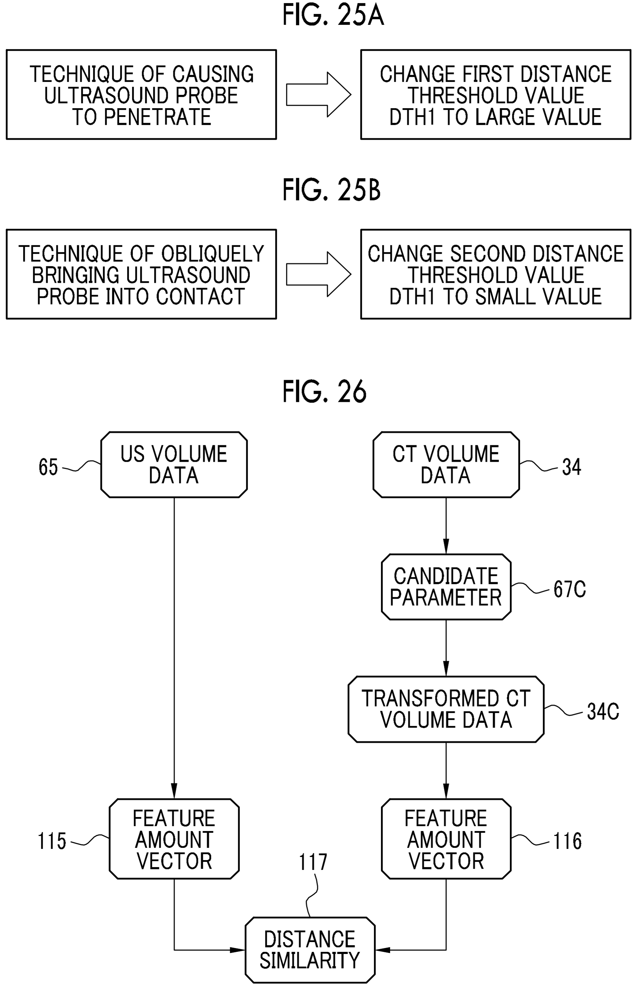

The constraint condition 52 may be changeable. As shown in FIG. 25A as an example, in a case of a technique of causing the ultrasound probe 10 to penetrate into the body, the first distance threshold value DTH1 of the first constraint condition 521 is changed to a large value. In addition, as shown in FIG. 25B as an example, in a case of a technique of obliquely bringing the ultrasound probe 10 into contact with the body surface 12, the second distance threshold value DTH2 of the second constraint condition 522 is changed to a small value. The technique of causing the ultrasound probe 10 to penetrate into the body may be performed on the patient P having a fat body type. In addition, the technique of obliquely bringing the ultrasound probe 10 into contact with the body surface 12 may be performed in intercostal and subcostal scanning. In a case in which the constraint condition 52 is changeable as described above, the determination of whether or not the candidate parameter 67C satisfies the constraint condition 52 can be performed in accordance with various situations. The constraint condition 52 may be changed depending on the model of the ultrasound probe 10.

In a case in which the site to which the ultrasound probe 10 is brought into contact is known in advance, a constraint condition of the site may be set. For example, in a case in which the site to which the ultrasound probe 10 is brought into contact is the right intercostal space and the position of the point P1C transformed from the point P1 indicating the position of the ultrasound probe 10 by the candidate parameter 67C in the distance image 90 is not the right intercostal space, it is determined that the candidate parameter 67C does not satisfy the constraint condition, and the candidate parameter 67C is discarded. In this case, the information of the site is registered in advance in each pixel of the distance image 90. By doing so, it is possible to suppress the output of the clearly incorrect rigid transformation parameter 67.

In a case in which it is determined that all the candidate parameters 67C do not satisfy the constraint condition 52, at least one of the following processes may be executed. In the first process, the candidate parameter 67C having the highest accuracy is adopted as the rigid transformation parameter 67, and then a message or the like is displayed on the display screen 19 of the composite image 16 to notify the operator S or the like that the registration is not accurate. In the second process, a message or the like is displayed on the display screen 19 of the composite image 16 to notify the operator S or the like that there is a possibility that the CT volume data 34 is confused with that of a different patient P. In the third process, a message or the like prompting the assistant AS to re-capture the US image 13 is displayed on the display 21.

A nonlinear transformation parameter may be derived as the transformation parameter instead of the linear transformation parameter such as the rigid transformation parameter 67. An MRI image may be adopted as the three-dimensional image instead of the CT image 15. The first point group may be extracted from the US image 13 instead of the example US volume data 65. The similarity is not limited to the example inlier extraction result 94. As shown in FIG. 26 as an example, feature amount vectors 115 and 116 of the US volume data 65 and the transformed CT volume data 34C using the candidate parameter 67C may be derived, and a distance similarity 117 of the feature amount vectors 115 and 116 may be calculated. Alternatively, the US volume data 65 and the transformed CT volume data 34 using the candidate parameter 67C may be input to the trained model, and the similarity may be output from the trained model. The technology of the present disclosure may be applied to the registration of the US image 13 and the CT image 15 in the postoperative follow-up observation instead of the registration of the US image 13 and the CT image 15 during the operation.

In each of the above-described embodiments, for example, each process of each processing unit such as the acquisition unit 55, the generation unit 56, the RW controller 57, the extraction unit 58, the initial registration unit 59, the registration unit 60, the display controller 61, the distance image generation unit 80, the estimation unit 81, the selection unit 82, the derivation unit 83, the determination unit 84, the transformation unit 85, the inlier extraction unit 86, and the decision unit 87 is executed by any computer. Moreover, any computer may execute these processes by a processor as hardware, a program as software, or a combination thereof. In such a case, the processor is configured to execute various types of processing in each of the above-described embodiments in cooperation with the program, and may function as each unit or each means in each of the above-described embodiments. Further, the execution order of the processing by the processor is not limited to the above-described order and may be changed as appropriate. Any computer may be a general-purpose computer, a computer for specific use, a workstation, or another system capable of executing each processing.

The processor may be composed of one or a plurality of pieces of hardware, and types of hardware are not limited. For example, the processor may be configured by the example CPU 42, a micro processing unit (MPU), a programmable logic device such as a field programmable gate array (FPGA), a dedicated circuit for executing specific processing, such as an application specific integrated circuit (ASIC), a graphics processing unit (GPU), a neural processing unit (NPU), or hardware. In addition, the types of hardware may be a combination of different types of hardware. In a case in which the plurality of types of hardware are configured to execute one or a plurality of types of processing of a certain processor, the plurality of types of hardware may exist in devices physically separated from each other or may exist in the same device. In addition, in any of the embodiments, the order of each processing by the processor is not limited to the above order, and may be appropriately changed. The hardware is configured by an electric circuit (circuitry) in which circuit elements such as semiconductor elements are combined.

The program may be software such as firmware or a microcode. In addition, the program may be, for example, a program module group, and each function thereof may be realized by a processor configured to execute each function. The program may be a program code or a plurality of code segments stored in one or a plurality of non-transitory computer-readable media (for example, a storage medium or other storage). The program may be stored in a plurality of non-transitory computer-readable media existing in devices physically separated from each other. The program code or the code segments may represent any combination of a procedure, a function, a subprogram, a routine, a subroutine, a module, a software package, a class, an instruction, a data structure, and a program statement. The program code or the code segments may be connected to other code segments or hardware circuits by transmitting and receiving information, data, an argument, a parameter, or content of a memory.

It is possible to understand the technology according to the following supplementary notes, based on the above description.

### [Supplementary Note 1]

An image processing apparatus including: a processor, in which the processor is configured to: acquire an ultrasound image of a subject and a three-dimensional image of the subject that is a target of registration of the ultrasound image; repeatedly select a plurality of reference points from a first point group of the ultrasound image and a plurality of target points from a second point group of the three-dimensional image a plurality of times; derive a candidate of a transformation parameter between the first point group and the second point group each time the reference points and the target points are selected; and determine whether the candidate satisfies a constraint condition related to a position and/or posture of an ultrasound probe at the time of capturing the ultrasound image.

### [Supplementary Note 2]

The image processing apparatus according to Supplementary Note 1, in which the processor is configured to: transform the three-dimensional image using the candidate determined to satisfy the constraint condition; calculate a similarity between the ultrasound image and the transformed three-dimensional image; and not perform the transformation and the calculation with respect to the candidate determined not to satisfy the constraint condition.

### [Supplementary Note 3]

The image processing apparatus according to Supplementary Note 2, in which the processor is configured to: transform the second point group using the candidate determined to satisfy the constraint condition; calculate a similarity between the first point group and the transformed second point group; and not perform the transformation and the calculation with respect to the candidate determined not to satisfy the constraint condition.

### [Supplementary Note 4]

The image processing apparatus according to Supplementary Note 3, in which the processor is configured to: transform the second point group using the candidate determined to satisfy the constraint condition; extract, based on the first point group and the transformed second point group, a point that is not an outlier from among the first point group; and not perform the transformation and the extraction with respect to the candidate determined not to satisfy the constraint condition.

### [Supplementary Note 5]

The image processing apparatus according to any one of Supplementary Notes 2 to 4, in which the processor is configured to adopt, among a plurality of the candidates determined to satisfy the constraint condition, a candidate having the highest similarity as the transformation parameter.

### [Supplementary Note 6]

The image processing apparatus according to any one of Supplementary Notes 1 to 5, in which the first point group and the second point group are point groups related to an organ and/or a blood vessel depicted in the ultrasound image and the three-dimensional image.

### [Supplementary Note 7]

The image processing apparatus according to Supplementary Note 6, in which the processor is configured to: determine a provisional transformation parameter using the first point group and the second point group related to the blood vessel; determine whether an accuracy of the provisional transformation parameter satisfies an accuracy condition; adopt the provisional transformation parameter as the transformation parameter in a case where the accuracy of the provisional transformation parameter is determined to satisfy the accuracy condition; and determine the transformation parameters using the first point group and the second point group related to the organ in a case where the accuracy of the provisional transformation parameter is determined not to satisfy the accuracy condition.

### [Supplementary Note 8]

The image processing apparatus according to any one of Supplementary Notes 1 to 7, in which the processor is configured to: determine whether the candidate satisfies a first constraint condition that is the constraint condition related to the position; and determine whether the candidate satisfies a second constraint condition that is the constraint condition related to the posture.

### [Supplementary Note 9]

The image processing apparatus according to Supplementary Note 8, in which the first constraint condition is a condition related to a distance between the position and a body surface of the subject in the three-dimensional image.

### [Supplementary Note 10]

The image processing apparatus according to Supplementary Note 8 or 9, in which the second constraint condition is a condition related to an orientation of the ultrasound probe with respect to the body surface of the subject in the three-dimensional image.

### [Supplementary Note 11]

The image processing apparatus according to any one of Supplementary Notes 1 to 10, in which the processor is configured to: generate a distance image representing a distance from a body surface of the subject from the three-dimensional image; and determine whether the candidate satisfies the constraint condition using the distance image.

### [Supplementary Note 12]

The image processing apparatus according to any one of Supplementary Notes 1 to 11, in which the constraint condition is changeable.

In the technology of the present disclosure, the above-described various embodiments and/or various modification examples may be combined with each other as appropriate. In addition, it goes without saying that the present disclosure is not limited to each of the embodiments described above, various configurations can be adopted as long as the configuration does not deviate from the gist. Furthermore, the technology of the present disclosure extends to a storage medium that non-transitorily stores the program, and a computer program product including the program, in addition to the program.

The described contents and illustrated contents shown above are detailed descriptions of the parts related to the technology of the present disclosure, and are merely an example of the technology of the present disclosure. For example, the above description of the configuration, the function, the operation, and the effect are the description of examples of the configuration, the function, the operation, and the effect of the parts according to the technology of the present disclosure. Accordingly, it goes without saying that unnecessary parts may be deleted, new elements may be added, or replacements may be made with respect to the above-described contents and the above-shown contents within a range that does not deviate from the gist of the technology of the present disclosure. Further, in order to avoid complications and facilitate understanding of the parts related to the technology of the present disclosure, descriptions of common general knowledge and the like that do not require special descriptions for enabling the implementation according to the technology of the present disclosure are omitted, in the described contents and illustrated contents shown above.

In the present specification, the term "A and/or B" is synonymous with the term "at least one of A or B". That is, "A and/or B" means that it may be only A, only B, or a combination of A and B. In addition, in the present specification, the same approach as "A and/or B" is applied to a case in which three or more matters are represented by connecting the matters with "and/or".

All documents, patent applications, and technical standards described in the present specification are incorporated by reference into the present specification to the same extent as in a case where the individual documents, patent applications, and technical standards were specifically and individually indicated to be incorporated by reference.

### Explanation of References

10: ultrasound probe
11: table
12: body surface
13: ultrasound image (US image)
13B: blood vessel extraction US image
13L: liver extraction US image
14: CT apparatus
15: CT image
15B: blood vessel extraction CT image
15L: liver extraction CT image
16: composite image
17: electrode needle
18: radio wave generation apparatus
19: display screen
20: apparatus main body
21: display
22: ultrasound diagnostic apparatus
25: probe position detection system
26: magnetic transmitter
27: magnetic position detection device
30: echo signal
31: magnetic position sensor
32: detection result
33: DICOM server
34: CT volume data
34C: transformed CT volume data
40: storage
41: memory
42: CPU
43: communication unit
44: bus line
50: operation program
51: extraction model
52: constraint condition
55: acquisition unit
56: generation unit
57: read and write controller (RW controller)
58: extraction unit
59: initial registration unit
60: registration unit
61: display controller
65: US volume data
66: extraction result
67: rigid transformation parameter
67C: candidate of rigid transformation parameter (candidate parameter)
67T: provisional rigid transformation parameter
70: blood vessel extraction model for US image
71: liver extraction model for US image
72: blood vessel extraction model for CT image
73: liver extraction model for CT image
75: first point group data
76: second point group data
76C: transformed second point group data
80: distance image generation unit
81: estimation unit
82: selection unit
83: derivation unit
84: determination unit
85: transformation unit
86: inlier extraction unit
87: decision unit
90: distance image
91: estimation result
92: selection result
93: determination result
94: inlier extraction result
100: reference point data
101: target point data
105: table
110: accuracy condition
115, 116: feature amount vector
117: distance similarity
521: first constraint condition
522: second constraint condition
ΔD: difference
AS: assistant
DP1C: distance from body surface of point P1C
DP2C: distance from body surface of point P2C
DTH1: first distance threshold value
DTH2: second distance threshold value
IR: inlier rate
P: patient
P1, P2, P1C, P2C: point
RTH: rate threshold value
S: operator
ST10, ST11, ST12, ST13, ST20, ST21, ST22, ST23, ST24, ST25, ST26, ST27, ST30, ST31, ST32, ST33, ST34, ST35, ST36, ST100, ST110, ST120, ST130, ST140, ST150, ST201, ST202, ST203, ST204: step
TL: target portion

## Claims

1. An image processing apparatus comprising:
a processor (42),
wherein the processor is configured to:
acquire an ultrasound image (13) of a subject and a three-dimensional image of the subject that is a target of registration of the ultrasound image (13);
repeatedly select a plurality of reference points from a first point group of the ultrasound image (13) and a plurality of target points from a second point group of the three-dimensional image a plurality of times;
derive a candidate of a transformation parameter between the first point group and the second point group each time the reference points and the target points are selected; and
determine whether the candidate satisfies a constraint condition related to a position and/or posture of an ultrasound probe (10) at the time of capturing the ultrasound image (13).

2. The image processing apparatus according to claim 1,
wherein the processor is configured to:
transform the three-dimensional image using the candidate determined to satisfy the constraint condition;
calculate a similarity between the ultrasound image (13) and the transformed three-dimensional image; and
not perform the transformation and the calculation with respect to the candidate determined not to satisfy the constraint condition.

3. The image processing apparatus according to claim 2,
wherein the processor is configured to:
transform the second point group using the candidate determined to satisfy the constraint condition;
calculate a similarity between the first point group and the transformed second point group; and
not perform the transformation and the calculation with respect to the candidate determined not to satisfy the constraint condition.

4. The image processing apparatus according to claim 3,
wherein the processor is configured to:
transform the second point group using the candidate determined to satisfy the constraint condition;
extract, based on the first point group and the transformed second point group, a point that is not an outlier from among the first point group; and
not perform the transformation and the extraction with respect to the candidate determined not to satisfy the constraint condition.

5. The image processing apparatus according to claim 2,
wherein the processor is configured to adopt, among a plurality of the candidates determined to satisfy the constraint condition, a candidate having the highest similarity as the transformation parameter.

6. The image processing apparatus according to claim 1,
wherein the first point group and the second point group are point groups related to an organ and/or a blood vessel depicted in the ultrasound image (13) and the three-dimensional image.

7. The image processing apparatus according to claim 6,
wherein the processor is configured to:
determine a provisional transformation parameter using the first point group and the second point group related to the blood vessel;
determine whether an accuracy of the provisional transformation parameter satisfies an accuracy condition (110);
adopt the provisional transformation parameter as the transformation parameter in a case where the accuracy of the provisional transformation parameter is determined to satisfy the accuracy condition (110); and
determine the transformation parameters using the first point group and the second point group related to the organ in a case where the accuracy of the provisional transformation parameter is determined not to satisfy the accuracy condition (110).

8. The image processing apparatus according to claim 1,
wherein the processor is configured to:
determine whether the candidate satisfies a first constraint condition that is the constraint condition related to the position; and
determine whether the candidate satisfies a second constraint condition that is the constraint condition related to the posture.

9. The image processing apparatus according to claim 8,
wherein the first constraint condition is a condition related to a distance between the position and a body surface (12) of the subject in the three-dimensional image.

10. The image processing apparatus according to claim 8,
wherein the second constraint condition is a condition related to an orientation of the ultrasound probe (10) with respect to the body surface (12) of the subject in the three-dimensional image.

11. The image processing apparatus according to claim 1,
wherein the processor is configured to:
generate a distance image (90) representing a distance from a body surface (12) of the subject from the three-dimensional image; and
determine whether the candidate satisfies the constraint condition using the distance image (90).

12. The image processing apparatus according to claim 1,
wherein the constraint condition is changeable.

13. An operation method of an image processing apparatus comprising:
acquiring an ultrasound image (13) of a subject and a three-dimensional image of the subject that is a target of registration of the ultrasound image (13);
repeatedly selecting a plurality of reference points from a first point group of the ultrasound image (13) and a plurality of target points from a second point group of the three-dimensional image a plurality of times;
deriving a candidate of a transformation parameter between the first point group and the second point group each time the reference points and the target points are selected; and
determining whether the candidate satisfies a constraint condition related to a position and/or posture of an ultrasound probe (10) at the time of capturing the ultrasound image (13).

14. A computer-readable storage medium storing an operation program of an image processing apparatus, the operation program causing a computer to execute a process comprising:
acquiring an ultrasound image (13) of a subject and a three-dimensional image of the subject that is a target of registration of the ultrasound image (13);
repeatedly selecting a plurality of reference points from a first point group of the ultrasound image (13) and a plurality of target points from a second point group of the three-dimensional image a plurality of times;
deriving a candidate of a transformation parameter between the first point group and the second point group each time the reference points and the target points are selected; and determining whether the candidate satisfies a constraint condition related to a position and/or posture of an ultrasound probe (10) at the time of capturing the ultrasound image (13).
